# EUROPEAN PATENT SPECIFICATION

(11) **EP 3 443 892 B1**
(45) Date of publication and mention of the grant of the patent: **25.09.2024**
(21) Application number: 17782235.0
(22) Date of filing: 28.03.2017
(51) Int. Cl.: A61B 5/02, A61B 5/024, A61B 5/00

(54) **PULSE WAVE DETECTION DEVICE AND BIOLOGICAL INFORMATION MEASURING DEVICE**
PULSWELLENDETEKTIONSVORRICHTUNG UND VORRICHTUNG ZUR MESSUNG BIOLOGISCHER INFORMATIONEN
DISPOSITIF DE DÉTECTION D'ONDES PULSATILES ET DISPOSITIF DE MESURE D'INFORMATIONS BIOLOGIQUES

(30) Priority: 13.04.2016 JP 2016080322
(43) Date of publication of application: 20.02.2019
(73) Proprietor: Omron Healthcare Co., Ltd., Muko-shi, Kyoto 617-0002 (JP)
(72) Inventor: FUJITA, Reiji, Muko-shi Kyoto 617-0002 (JP); YAMASHITA, Shingo, Muko-shi Kyoto 617-0002 (JP)
(74) Representative: Isarpatent
(86) International application number: PCT/JP2017/012697
(87) International publication number: WO 2017/179417

(56) References cited:
- JP-A- 2004 129 979
- JP-A- 2005 324 004
- JP-A- 2014 018 357
- JP-A- 2014 018 357
- US-A1- 2005 234 351

## Description

### Technical Field

The present invention relates to a pulse wave detector and a biometric information measurement device

### Background Art

A biometric information measurement device is known that, in a state where a sensor is directly contacted with a living body portion through which an artery such as the radial artery in the wrist passes, can measure biometric information such as the pulse rate or the blood pressure by using information detected by the sensor (for example, see Patent Literatures 1 and 2).

A blood pressure measurement device disclosed in Patent Literature 1 calculates a blood pressure value by using a cuff in a portion different from a living body portion with which a pressure sensor is contacted, and produces calibration data from the calculated blood pressure value. A pressure pulse wave detected by a pressure pulse wave sensor which is attached to the wrist is calibrated by using the calibration data, thereby calculating the blood pressure value for every pulse. The pressure pulse wave sensor has a configuration where the sensor is accommodated in a housing, and the housing is secured to the wrist with a replaceable band.

A biometric information measurement device disclosed in Patent Literature 2 is of the wristwatch type. In the device, a housing accommodating a sensor is secured to the wrist with a replaceable band.

Patent Literature 3 describes a pulse wave detector including a housing and a band for attaching the housing to a wrist, wherein the band is attached to engagement portions provided at opposite side ends of the housing.

Patent Literature 4 discloses a pulse wave detector comprising a housing and a band, wherein a band insertion portion having an attachment hole is provided at a left side end portion of the housing. An end edge portion of a second band portion of the band can be inserted into said attachment hole. Further, a band attachment portion is provided at a right side end portion of the housing, wherein the band attachment portion has an attachment portion to which a left side end portion of the second band portion is attached.

### Citation List

### Patent Literature

Patent Literature 1: JP-A-H5-3858
Patent Literature 2: JP-A-2005-324004
Patent Literature 3: US 2005/0234351 A1
Patent Literature 4: JP 2014 018357 A

### Summary of Invention

### Technical Problem

A pulse wave detector which detects a pulse wave from an artery in the wrist while being attached to the wrist is required to have superior attachability to the wrist, and accuracy of positional alignment between a pulse wave detecting section and the artery. In a band structure such as disclosed in Patent Literature 1 and Patent Literature 2, however, a band is singly wound around the wrist of the measurement subject. Therefore, the stability during attachment is low, and there is a possibility that positional displacement of the pressure detecting section may occur after attachment.

The present invention has been made in view of the above circumstances. It is an object of the present invention to provide a pulse wave detector which can accurately detect a pulse wave while realizing superior attachability and preventing positional displacement of a pulse wave detecting section with respect to the radial artery from occurring, and also a biometric information measurement device including the detector.

### Means for Solving Problems

The present invention provides a pulse wave detector in accordance with claim 1. The pulse wave detector according to the present invention is used while being attached to a wrist of a measurement subject and includes: a body portion which includes a detecting section disposed in a position which is opposed to a radial artery of the measurement subject and configured to detect a pulse wave from the radial artery of the measurement subject; a band which is configured to be wound around the wrist in a state where the band is engaged with the body portion, so as to maintain a state where the detecting section is opposed to the radial artery; a basal-end engaging portion which is disposed on a first end portion of the body portion in a circumferential direction of the wrist and on a side of a surface opposite to a detection surface in the body portion, and with which a basal end portion of the band is engaged; an arbitrary-position engaging portion which is disposed on a second end portion of the body portion in the circumferential direction and on the detection surface, and with which an arbitrary position between the basal end portion engaged with the basal-end engaging portion and a tip end portion of the band is engaged in a folded back state; and engagement members which are configured to detachably engage the tip end portion with the band in a state where the band which is folded back in the arbitrary-position engaging portion and the basal end portion engaged with the basal-end engaging portion overlap with each other. The basal-end engaging portion is configured by a first member which is erected from the first end portion of the body portion and which has a Π-shape, and a first hole portion is formed between the first member and the first end portion of the body portion. The arbitrary-position engaging portion is configured by a second member which is erected from the second end portion of the body portion and which has a Π-shape, and a second hole portion is formed between the second member and the second end portion of the body portion. The engagement members are formed on surfaces of the basal end portion and the tip end portion of the band.

A biometric information measurement device according to the present invention includes the above pulse wave detector; and a biometric information calculating section which is configured to calculate biometric information based on the pulse wave detected by the detecting section.

### Advantageous Effects of Invention

According to the present invention, it is possible to provide a pulse wave detector which can accurately detect a pulse wave while realizing superior attachability and preventing positional displacement of a pulse wave detecting section with respect to the radial artery from occurring, and also a biometric information measurement device including the detector.

### Brief Description of Drawings

Fig. 1 is a perspective view schematically showing the external configuration of a biometric information measurement device 10 of a first embodiment of the present invention.
Fig. 2 is a side view of the biometric information measurement device 10 shown in Fig. 1, as seen from the side of the elbow of the left hand of a measurement subject.
Fig. 3 is a view of the vicinity of a first engaging portion 29 of the biometric information measurement device 10 shown in Fig. 2, as seen from the side of a display surface 23 of a housing 20.
Fig. 4 is a side view of a biometric information measurement device 11 which is a modification of the biometric information measurement device 10 shown in Fig. 1, as seen from the side of the elbow of the left hand of the measurement subject.
Fig. 5 is a side view of a biometric information measurement device 12 which does not form part of the present invention and which is a modification of the biometric information measurement device 10 shown in Fig. 1, as seen from the side of the elbow of the left hand of the measurement subject.
Fig. 6 is a side view of a biometric information measurement device 13 which does not form part of the present invention and which is a modification of the biometric information measurement device 10 shown in Fig. 1, as seen from the side of the elbow of the left hand of the measurement subject.
Fig. 7 is a side view of a biometric information measurement device 14 which is a modification of the biometric information measurement device 10 shown in Fig. 1, as seen from the side of the fingers of the left hand of the measurement subject.
Fig. 8 is a side view of a biometric information measurement device 15 which does not form part of the present invention and which is a modification of the biometric information measurement device 10 shown in Fig. 1, as seen from the side of the elbow of the left hand of the measurement subject.
Fig. 9 is a view showing a modification of a body portion of the biometric information measurement device 10 shown in Fig. 1.

### Description of Embodiments

Hereinafter, embodiments of the present invention will be described with reference to the drawings.

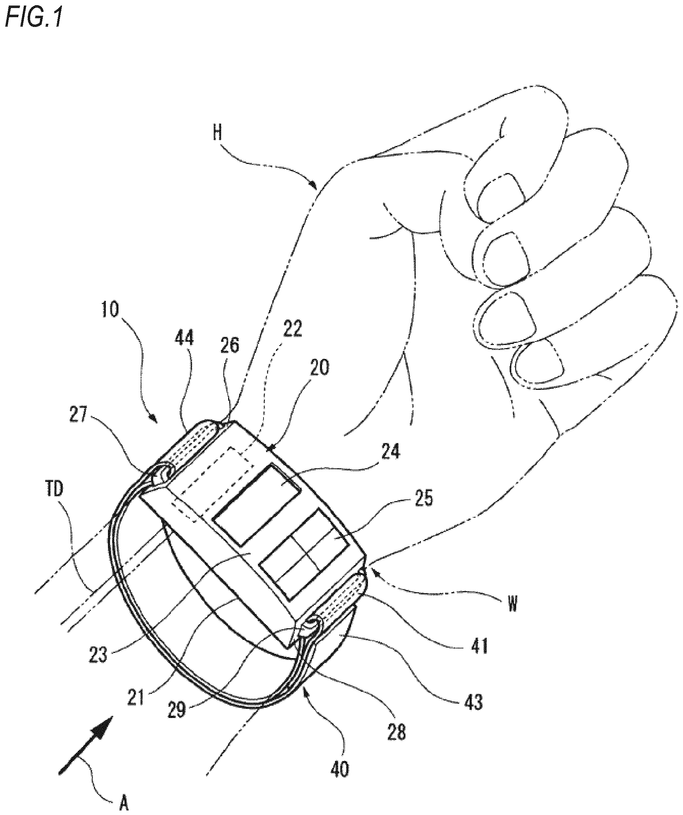

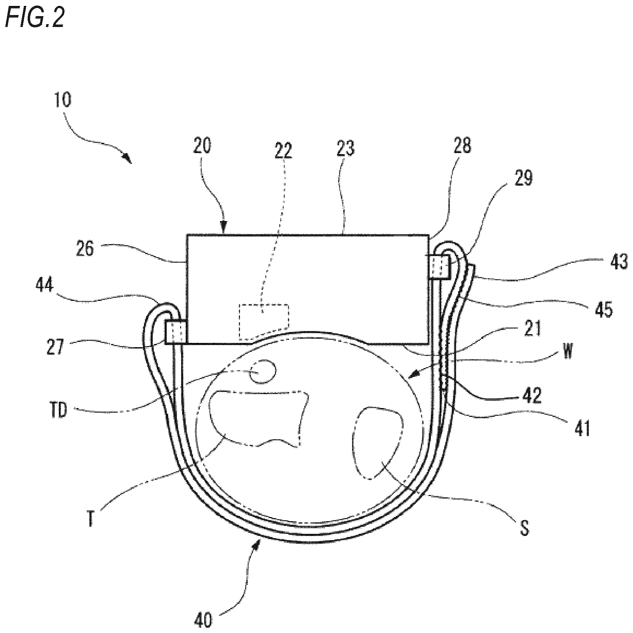

Fig. 1 is a perspective view schematically showing the external configuration of a biometric information measurement device 10 of a first embodiment of the present invention. As shown in Fig. 1, the biometric information measurement device 10 is used while being attached to the wrist W of the left hand H of a measurement subject. Fig. 2 is a side view of the biometric information measurement device 10 shown in Fig. 1, as seen from the side (the direction A in Fig. 1) of the elbow of the left hand H of the measurement subject.

The biometric information measurement device 10 has: a housing 20 which constitutes a body portion including a pulse wave detecting section 22 that can detect a pulse wave (a pressure pulse wave or a volume pulse wave) from the radial artery TD extending along the radius T in the wrist W of the measurement subject, and which is formed of a metal or a resin; a band 40 which is engaged with the housing 20.

The pulse wave detecting section 22 may have a known configuration. For example, the pulse wave detecting section 22 has a pressure sensor, and a mechanism which presses it against the skin, and detects a pressure pulse wave by using the pressure sensor. Alternatively, the pulse wave detecting section 22 has a photoelectric sensor, and detects a volume pulse wave from a signal detected by the photoelectric sensor.

In the example of Fig. 1, the housing 20 is a member having an approximately box-like shape, and includes the pulse wave detecting section 22, and a biometric information calculating section (not shown) which calculates biometric information such as the heart rate, the pulse rate, or the blood pressure value based on the pulse wave detected by the pulse wave detecting section 22.

The biometric information calculating section may be disposed in an apparatus other than the biometric information measurement device 10. Namely, the housing 20 of the biometric information measurement device 10 may need to have at least the pulse wave detecting section 22. In this case, the biometric information measurement device 10 functions as the pulse wave detector.

The surface (the surface opposed to the wrist W) of the housing 20 which, in the case where the biometric information measurement device 10 is attached to the wrist W, is opposed to the wrist W constitutes a detection surface 21. The detection surface 21 may have an approximately flat shape, or a part or the whole of the surface may be arcuate so as to extend along the outer shape of the wrist W. The pulse wave detecting section 22 is disposed in a position which is opposed to the radial artery TD in a state where the detection surface 21 is opposed to the wrist W.

In the housing 20, the surface (the opposite surface) opposite to the detection surface 21 constitutes a display surface 23. In the display surface 23, a displaying portion 24 configured by a liquid crystal display device or the like for displaying measurement results, and an operating section 25 for operating the biometric information measurement device 10 are disposed. The positions where the displaying portion 24 and the operating section 25 are respectively disposed are not limited to those shown in Fig. 1. A configuration where the displaying portion 24 is omitted may be employed.

A first engaging portion 29 is disposed on a first end portion 28 (in the example of Figs. 1 and 2, an end surface of the housing 20 on the side of the ulna S) which is one of end portions of the housing 20 on the side of the ulna S in the circumferential direction of the wrist W. The first engaging portion 29 is disposed on the first end portion 28, and on the side of the display surface 23. Namely, the first engaging portion 29 is disposed on a part which is in the first end portion 28, and which is on the side that is close to the display surface 23 in the case where the housing 20 is divided into halves in a direction perpendicular to the display surface 23.

A second engaging portion 27 is disposed on a second end portion 26 (in the example of Figs. 1 and 2, an end surface of the housing 20 on the side of the radius T) which is one of end portions of the housing 20 on the side of the radius T in the circumferential direction of the wrist W.

The first engaging portion 29 and the second engaging portion 27 are used for causing the band 40 to engage with the housing 20. Each of the first engaging portion 29 and the second engaging portion 27 has a shape that has a hole portion into which the band 40 can be inserted.

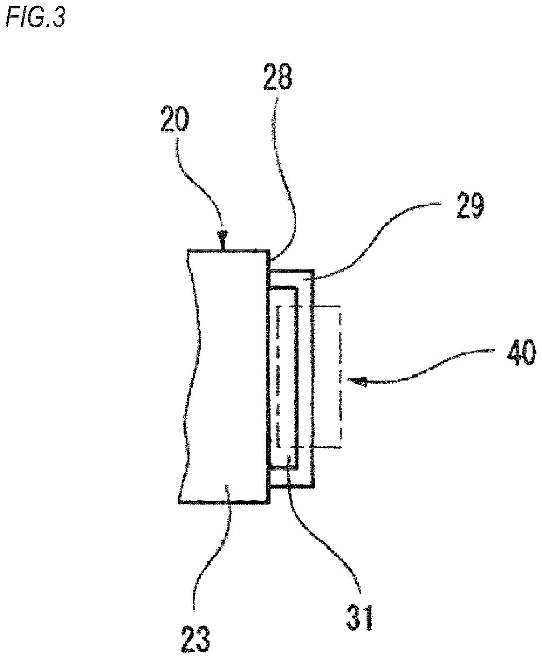

Fig. 3 is a view of the vicinity of the first engaging portion 29 of the biometric information measurement device 10 shown in Fig. 2, as seen from the side of the display surface 23 of the housing 20.

As shown in Fig. 3, the first engaging portion 29 is configured by a member which is erected from the first end portion 28 of the housing 20, and which has a shape approximately similar to a Greek character Π, and a hole portion 31 is formed between this member and the first end portion 28 of the housing 20. The band 40 can be inserted into the hole portion 31. The second engaging portion 27 is configured in a similar manner to the first engaging portion 29.

The first engaging portion 29 is disposed on the first end portion 28, and is disposed on the display surface 23 of the first end portion 28.

The second engaging portion 27 is be disposed on the second end portion 26, and is disposed on the detection surface 21 of the second end portion 26.

The band 40 is configured to be wound around the wrist W in a state where one end is engaged with the housing 20 (routed so as to form a state where the wrist W is sandwiched between the band 40 and the housing 20), so as to maintain the state where the pulse wave detecting section 22 is opposed to the radial artery TD.

For example, the band 40 is a strip-like member which is lower in rigidity than the housing 20. As the material of the band 40, for example, cloth, leather, rubber, thin resin, or the like may be used. Therefore, the band 40 can be easily folded back.

A basal end portion 41 which constitutes one end of the band 40 in the longitudinal direction is detachably engaged with the first engaging portion 29 which is disposed on the first end portion 28.

Specifically, the basal end portion 41 of the band 40 is inserted into the hole portion 31 of the first engaging portion 29, in a direction from the side of the detection surface 21 toward the display surface 23. The basal end portion 41 which has passed through the hole portion 31 is folded back in an approximately U-like shape in a direction to be separated from the housing 20. A hook and loop fastener 42 is formed on the surface of the non-folded back part of the basal end portion 41 and directed in the direction opposite to the housing 20, and the surface of the folded back part of the basal end portion 41 and directed to the housing 20. Because of the hook and loop fastener 42, the folded back part of the basal end portion 41 is detachably engaged with the band 40.

The hook and loop fastener 42 is an engagement member for causing the basal end portion 41 to detachably engage with the band 40. Incidentally, a configuration may be employed where, in the state shown in Fig. 2, a convex portion or concave portion which is disposed on the surface of the folded back part of the basal end portion 41 and directed to the housing 20 is fitted into a concave portion or convex portion which is disposed on the surface of the non-folded back part of the basal end portion 41 and directed in the direction opposite to the housing 20, whereby the basal end portion 41 is detachably engaged with the band 40.

In this case, the concave portion and the convex portion constitute the engagement member. Alternatively, the non-folded back part and folded back part of the basal end portion 41 may be completely secured to each other by an adhesive agent, sewing, or the like. Namely, the basal end portion 41 may be non-detachably engaged with the first engaging portion 29.

An arbitrary position 44 between the basal end portion 41 of the band 40 which is engaged with the first engaging portion 29, and a tip end portion 43 which is the end portion that is opposite in the longitudinal direction to the basal end portion 41 of the band 40 is engaged in the state where the band is folded back in a direction to be separated from the housing 20, in the second engaging portion 27 that is disposed on the second end portion 26 of the housing 20.

Specifically, in the band 40 which is engaged with the first engaging portion 29, the tip end portion 43 is inserted into the hole portion of the second engaging portion 27 from the side of the detection surface 21 toward the display surface 23. The band 40 which is inserted into the hole portion of the second engaging portion 27 is folded back in the arbitrary position 44 in an approximately U-like shape in a direction to be separated from the housing 20. In the state where the tip end portion 43 of the folded back band 40 overlaps with the first end portion 28 in the circumferential direction of the wrist W, the tip end portion 43 is engaged with the basal end portion 41 with a hook and loop fastener 45 which is formed on the surfaces of the basal end portion 41 and tip end portion 43 of the band 40.

The hook and loop fastener 45 is an engagement member for causing the tip end portion 43 to detachably engage with the basal end portion 41.

Incidentally, a configuration may be employed where, in the state shown in Fig. 2, a convex portion or concave portion which is disposed on the surface of the tip end portion 43 and directed to the housing 20 is fitted into a concave portion or convex portion which is disposed on the surface of the basal end portion 41 and directed in the direction opposite to the housing 20, whereby the tip end portion 43 is detachably engaged with the basal end portion 41. In this case, the concave portion and the convex portion constitute the engagement member.

An example of a method of attaching the thus configured biometric information measurement device 10 to the wrist W will be described.

Firstly, the measurement subject makes the basal end portion 41 of the band 40 pass through the hole portion 31 of the first engaging portion 29 of the housing 20 from the side of the detection surface 21 toward the display surface 23. Then, the measurement subject makes the basal end portion 41 which has passed through the hole portion 31, to be folded back in the direction to be separated from the housing 20 to be engaged with the band 40 through the hook and loop fastener 42. As a result, the basal end portion 41 is engaged with the first engaging portion 29.

Next, the measurement subject makes the tip end portion 43 of the band 40 pass through the hole portion of the second engaging portion 27 of the housing 20 from the side of the detection surface 21 toward the display surface 23, and folds back the band 40 which has passed through the hole portion of the second engaging portion 27, in the direction to be separated from the housing 20. As a result, the arbitrary position 44 of the band 40 is engaged with the second engaging portion 27. In this state, the measurement subject makes the arm pass through a space interposed between a portion between the basal end portion 41 and arbitrary position 44 of the band 40, and the detection surface 21.

After the measurement subject positions the housing 20 so that the pulse wave detecting section 22 is opposed to the radial artery TD, then, the measurement subject wounds the tip end portion 43 of the band 40 around the wrist W, and pulls up the tip end portion 43 to the vicinity of the first end portion 28 while directing from the hand back side to the palm side, i.e., from the side of the detection surface 21 toward the display surface 23.

The measurement subject adjusts the force of pulling the tip end portion 43 of the band 40, so as to cause the housing 20 to be in close contact with the wrist W at an adequate pressure. In this state, the measurement subject makes the tip end portion 43 of the band 40 overlap with the basal end portion 41 engaged with the first engaging portion 29 to be engaged with the basal end portion 41 through the hook and loop fastener 42.

At this time, the tip end portion 43 of the band 40 is engaged in a state where the tip end is directed from the detection surface 21 of the housing 20 toward the display surface 23. In other words, the tip end portion 43 of the band 40 is engaged with the basal end portion 41 in a state where the position of the tip end of the tip end portion 43 in a direction in which the detection surface 21 and display surface 23 of the housing 20 are juxtaposed (the vertical direction in Fig. 2) is on the side of the display surface 23 with respect to the position of the detection surface 21 of the housing 20 in this direction.

According to the biometric information measurement device 10, as described above, the tip end portion 43 of the band 40 is engaged in the state where the tip end is directed from the side of the detection surface 21 of the housing 20 toward the display surface 23. Therefore, a force which is applied to the wrist W when the band 40 is pulled is facilitated to evenly act on the side of the thumb and on the side of the little finger, and it is possible to prevent positional displacement of the pulse wave detecting section 22 from being caused. The attachment of the biometric information measurement device 10 can be performed while the back of the hand on a table remains to be opposed to the table surface. Therefore, it is possible to prevent positional displacement of the pulse wave detecting section 22 due to a motion of raising the hand from the table, from occurring.

According to the biometric information measurement device 10, as shown in Fig. 2, the housing 20 is secured to the wrist W in the state where the band 40 is doubly wound around the wrist W. In this manner, the wrist W can be covered by the double portions of the band 40, and therefore it is possible to prevent positional displacement of the housing 20 from occurring after attachment of the biometric information measurement device 10.

During attachment of the biometric information measurement device 10, the band 40 can be fastened in the state where the wrist W is inserted into the space interposed between the housing 20 and the band 40, and the housing 20 is positioned. As compared with the case where the band 40 is singly wound, therefore, the biometric information measurement device 10 can be easily attached. Moreover, the accuracy of positioning the pulse wave detecting section 22 can be improved.

Preferably, a configuration may be employed where the friction coefficient of the surface of the band 40 opposite to the side which is in contact with the wrist W is smaller than that of the surface on the side which is in contact with the wrist W.

According to the configuration, when the band 40 which is folded back in the second engaging portion 27 is to be wound around the wrist W, the band 40 easily slips over a portion of the band 40 which has been already wound around the wrist W. Therefore, the fastening manner of the band 40 can be easily adjusted, and the attachability and the accuracy of positioning the pulse wave detecting section 22 can be improved. With respect to the surface which is in contact with the wrist W, moreover, the band 40 and the wrist W hardly slip over each other, and therefore it is possible to prevent positional displacement of the housing 20 from occurring after attachment of the biometric information measurement device 10.

The biometric information measurement device 10 has the configuration where the first engaging portion 29 is disposed on the first end portion 28 and on the side of the display surface 23. According to the configuration, when the wrist W on a table is turned from a state where, for example, the back of the hand is directed to the table surface, toward the side of the little finger, the first engaging portion 29 is hardly contacted with the table surface. Therefore, a superior usability is obtained, and positional displacement of the pulse wave detecting section 22 can be prevented from occurring.

The biometric information measurement device 10 has the configuration where the basal end portion 41 of the band 40 is detachably engaged with the first engaging portion 29. According to the configuration, the band 40 can be completely detached from the housing 20. In the case where the band 40 is contaminated with human sweat or external environment (dust, scratch, or the like), therefore, this situation can be dealt with by replacement of the band 40.

The diameter and shape of the wrist of the measurement subject are different among individuals. When the band 40 is replaceable, a configuration may be possible where, for example, a plurality of kinds of bands 40 are selectively used in accordance with the measurement subject. As a result, regardless of individual variations, it is possible to realize superior attachability.

The biometric information measurement device 10 has the configuration where the basal end portion 41 of the band 40 is engaged with the first engaging portion 29 in the state where the basal end portion is folded back to be separated from the housing 20. According to the configuration, a work of detaching the band 40 from the housing 20 is easily performed, and the usability can be improved.

The biometric information measurement device 10 has the configuration where the tip end portion 43 of the band 40 is engaged with the basal end portion 41 on the side of the ulna S in the wrist W. The measurement subject performs a work of routing the band 40 with using the right hand. Therefore, the engagement of the tip end portion 43 of the band 40 can be performed on the side of the ulna S where the right hand exists, whereby attachment of the biometric information measurement device 10 can be easily performed.

The biometric information measurement device 10 has the configuration where the tip end portion 43 of the band 40 is engaged with the basal end portion 41 with the hook and loop fastener 45. According to the configuration, the function of the hook and loop fastener 45 facilitates adjustment of the winding force of the band 40. Therefore, the attachability can be improved.

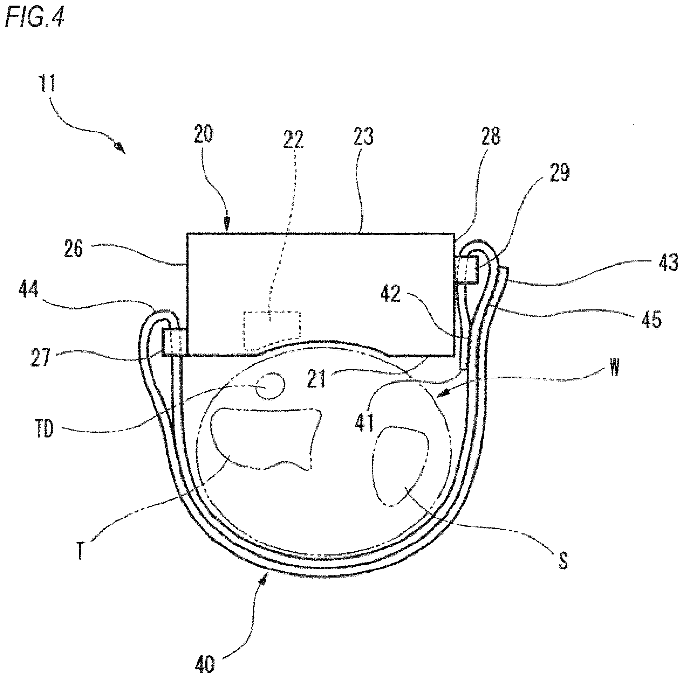

Fig. 4 is a side view of a biometric information measurement device 11 which is a modification of the biometric information measurement device 10 shown in Fig. 1, as seen from the side of the elbow of the left hand of the measurement subject. In Fig. 4, portions which are in common with the biometric information measurement device 10 are denoted by the same reference numerals, and duplicated description is omitted.

In the biometric information measurement device 11, the folding-back direction of the basal end portion 41 of the band 40 is opposite to that in the biometric information measurement device 10. The tip end portion 43 of the band 40 is engaged on the surface which is in the non-folded back part of the basal end portion 41, and which is directed in the direction opposite to the housing 20. The other configuration is identical with that of the biometric information measurement device 10.

Specifically, the basal end portion 41 of the band 40 of the biometric information measurement device 11 passes through the hole portion 31 of the first engaging portion 29 which is disposed on the first end portion 28 of the housing 20, from the side of the display surface 23 toward the detection surface 21. Then, the folded back part of the basal end portion 41 is engaged with the non-folded back part of the basal end portion 41 with the hook and loop fastener 42.

The tip end portion 43 of the band 40 of the biometric information measurement device 11 is engaged with the basal end portion 41 with the hook and loop fastener 45, at a position where the portion overlaps with the first end portion 28 in the circumferential direction of the wrist W.

According to the biometric information measurement device 11, the part of the basal end portion 41 with which the tip end portion 43 of the band 40 is engaged has an approximately flat surface. Therefore, a work of attaching or detaching the tip end portion 43 can be easily performed.

The biometric information measurement device 11 has the configuration where the basal end portion 41 of the band 40 is engaged with the first engaging portion 29 in a state where the basal end portion is folded back in a direction approaching the housing 20. When the engagement state of the tip end portion 43 with respect to the basal end portion 41 is to be released, therefore, the engagement state between the parts of the basal end portion 41 is hardly released. Consequently, the engagement state of the basal end portion 41 with respect to the first engaging portion 29 can be stably maintained.

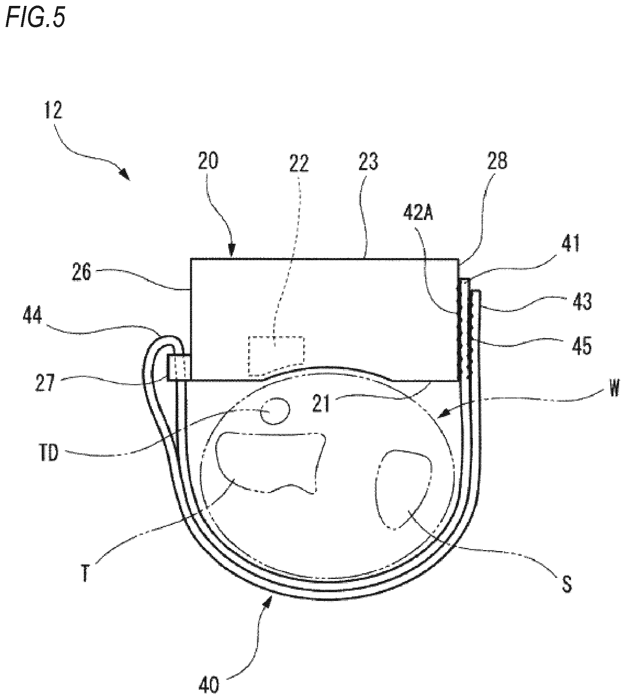

Fig. 5 is a side view of a biometric information measurement device 12 which is a modification of the biometric information measurement device 10 shown in Fig. 1, as seen from the side of the elbow of the left hand of the measurement subject. In Fig. 5, portions which are in common with the biometric information measurement device 10 are denoted by the same reference numerals, and duplicated description is omitted.

In the biometric information measurement device 12, the above-described first engaging portion 29 is not disposed on the first end portion 28 of the housing 20. Instead, the basal end portion 41 of the band 40 of the biometric information measurement device 12 is engaged with the first end portion 28 through a hook and loop fastener 42A. In the biometric information measurement device 12, the hook and loop fastener 42A which is formed on the first end portion 28 constitutes the first engaging portion.

The hook and loop fastener 42A is an engagement member for detachably engaging the basal end portion 41 to the first end portion 28. Instead of the hook and loop fastener 42A, the above-described concavo-convex structure may be employed.

The tip end portion 43 of the band 40 of the biometric information measurement device 12 is detachably engaged through the hook and loop fastener 45 with the surface of the basal end portion 41 which is engaged with the first end portion 28, the surface being directed in the direction opposite to the housing 20.

The other configuration is similar to that of the biometric information measurement device 10.

According to the biometric information measurement device 12, the part of the basal end portion 41 with which the tip end portion 43 of the band 40 is engaged has an approximately flat surface. Therefore, a work of attaching or detaching the tip end portion 43 can be easily performed.

In the biometric information measurement device 12, alternatively, the basal end portion 41 may be non-detachably engaged with the first end portion 28 by adhesion, screws, or the like. According to the configuration, the engagement state of the basal end portion 41 can be stably maintained.

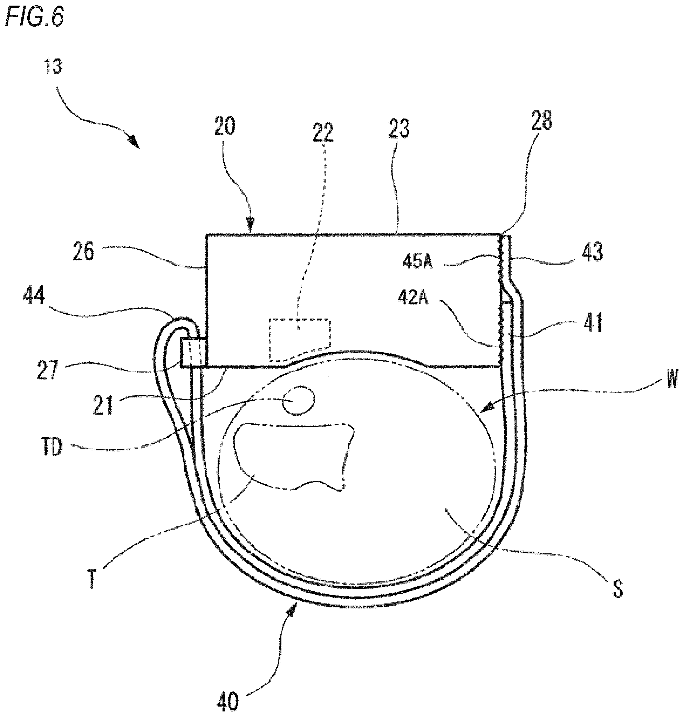

Fig. 6 is a side view of a biometric information measurement device 13 which is a modification of the biometric information measurement device 10 shown in Fig. 1, as seen from the side of the elbow of the left hand of the measurement subject. In Fig. 6, portions which are in common with those of the biometric information measurement device 12 are denoted by the same reference numerals, and duplicated description is omitted.

The biometric information measurement device 13 is configured in the same manner as the biometric information measurement device 12 shown in Fig. 5 except that the tip end portion 43 of the band 40 is engaged with the first end portion 28 of the housing 20 with a hook and loop fastener 45A.

The tip end portion 43 of the band 40 of the biometric information measurement device 13 is engaged with the first end portion 28 with the hook and loop fastener 45A in a state where the tip end portion overlaps with the basal end portion 41 in the circumferential direction of the wrist W.

The hook and loop fastener 45A is an engagement member for causing the tip end portion 43 to detachably engage with the first end portion 28. Instead of the hook and loop fastener 45A, the above-described concavo-convex structure may be employed.

The biometric information measurement device 13 can attain effects similar to those of the biometric information measurement device 12. According to the biometric information measurement device 13, when the engagement state of the tip end portion 43 with respect to the housing 20 is to be cancelled, it is possible to easily prevent the engagement state between the basal end portion 41 and the housing 20 from being cancelled.

The biometric information measurement device 13 may be configured so that the hook and loop fastener 45A is extended onto the display surface 23, and the tip end portion 43 of the band 40 is engaged with the housing 20 by two surfaces or the end surface of the housing 20 and the display surface 23. According to the configuration, it is possible to prevent the vicinity of the tip end of the tip end portion 43 from being in a free state, and the beauty of the device can be improved. Moreover, it is possible to prevent the tip end portion 43 of the band 40 from peeling off after attachment of the biometric information measurement device 10.

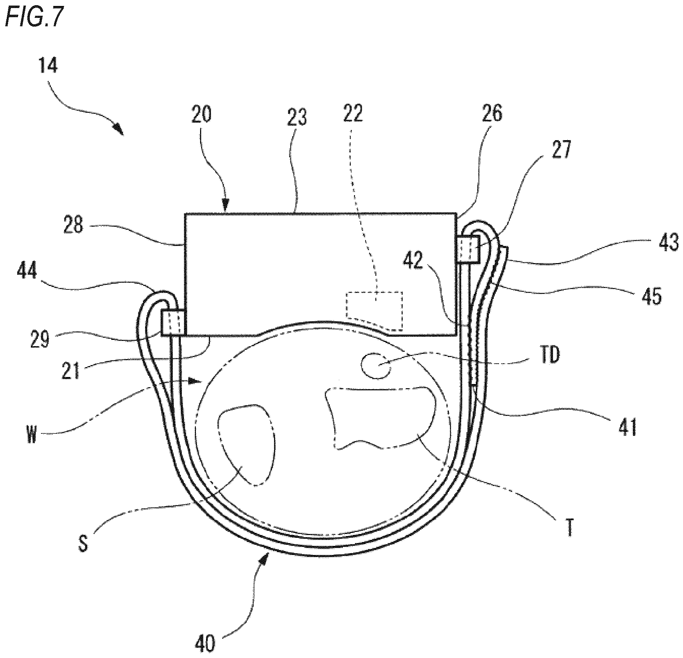

Fig. 7 is a side view of a biometric information measurement device 14 which is a modification of the biometric information measurement device 10 shown in Fig. 1, as seen from the side of the fingers of the left hand of the measurement subject. In Fig. 7, portions which are in common with the biometric information measurement device 10 are denoted by the same reference numerals, and duplicated description is omitted.

In the biometric information measurement device 14, the band 40, which is attached to the biometric information measurement device 10, is attached to the housing 20 in a laterally reversed manner.

In the biometric information measurement device 14, namely, the basal end portion 41 of the band 40 is folded back in the second engaging portion 27 disposed on the second end portion 26, and engaged with the hook and loop fastener 42. Moreover, an arbitrary position 44 in the longitudinal direction of the band 40 is folded back in the first engaging portion 29 disposed on the first end portion 28. Then, the tip end portion 43 of the band 40 is engaged by the hook and loop fastener 45 with the basal end portion 41 engaged with the second engaging portion 27.

The biometric information measurement device 14 can attain effects similar to those of the biometric information measurement device 10. In the biometric information measurement device 14, it is preferable that, similarly with the biometric information measurement device 10, the first engaging portion 29 on the first end portion 28 is disposed on the side of the display surface 23. According to the configuration, it is possible to improve the usability of the biometric information measurement device 14.

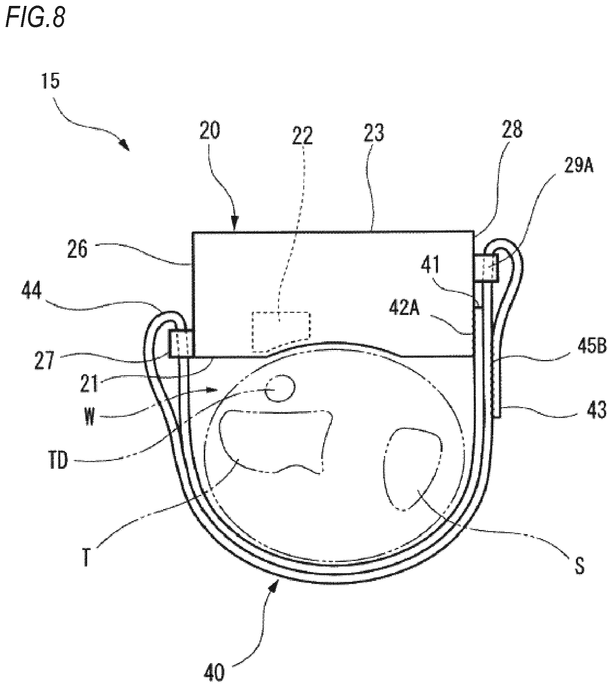

Fig. 8 is a side view of a biometric information measurement device 15 which is a modification of the biometric information measurement device 10 shown in Fig. 1, as seen from the side of the elbow of the left hand of the measurement subject. In Fig. 8, portions which are in common with the biometric information measurement device 13 are denoted by the same reference numerals, and duplicated description is omitted.

The biometric information measurement device 15 has a configuration where the tip end portion 43 of the band 40 is folded back in a third engaging portion 29A in a direction to be separated from the housing 20, and the folded back part of the tip end portion 43 is engaged with the band 40 by a hook and loop fastener 45B. The other configuration is identical with that of the biometric information measurement device 13.

The third engaging portion 29A has the same configuration as the first engaging portion 29, and is configured by an approximately Π-shaped member. The tip end portion 43 of the band 40 of the biometric information measurement device 15 is inserted into the hole portion of the third engaging portion 29A from the side of the detection surface 21 toward the display surface 23, and the tip end portion 43 which has passed through the hole portion is folded back in a direction to be separated from the housing 20. Then, the folded back tip end portion 43 is engaged with the band 40 through the hook and loop fastener 45B which is formed on the band 40.

The hook and loop fastener 45B is an engagement member for causing the tip end portion 43 to detachably engage with the band 40. Instead of the hook and loop fastener 45B, the above-described concavo-convex structure may be employed.

The biometric information measurement device 15 can attain effects similar to those of the configuration of the biometric information measurement device 10. The biometric information measurement device 15 has the configuration where the tip end portion 43 is folded back in the third engaging portion 29A and then engaged, and therefore the band 40 is fastened more firmly.

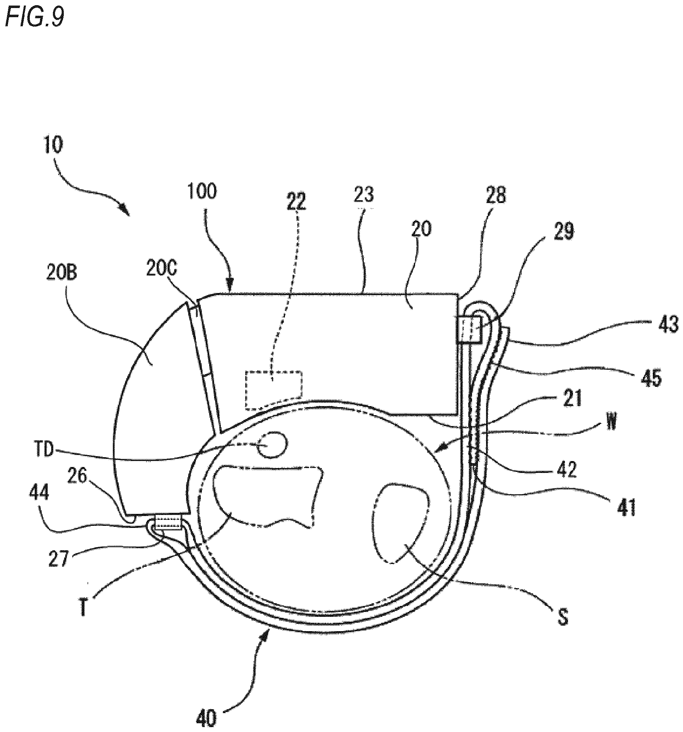

Although the examples in which the body portion of the biometric information measurement device 10 is configured by the single housing 20 have been described, the configuration is not limited thereto. Fig. 9 is a view showing a modification of the body portion of the biometric information measurement device 10. In Fig. 9, configurations which are identical with those of Fig. 2 are denoted by the same reference numerals.

The body portion 100 of the biometric information measurement device 10 shown in Fig. 9 is configured by: the above-described housing 20; a housing 20B; and a coupling portion 20C which swingably couples together the housing 20 and the housing 20B, such as a hinge. In the example of Fig. 9, for example, the housing 20B accommodates batteries or the like for driving the biometric information measurement device 10.

The body portion 100 has a longitudinal shape which extends along the circumferential direction of the wrist, as a whole, and a shape which is opened at least on the side of the ulna S in the wrist. The body portion 100 has a configuration where, in a state where the body portion is attached to the wrist by the band 40, the portion between the end portions in the circumferential direction of the wrist does not cover the wrist at a degree that a part of the band 40 is in contact with the wrist.

In the example of Fig. 9, an end portion which is one of the end portions of the housing 20B on the side opposite to the housing 20 in the circumferential direction of the wrist constitutes the above-described second end portion 26, and the second engaging portion 27 is formed on the second end portion 26.

The above-disclosed embodiment should be considered in all respects to be illustrative and not restrictive. The scope of the present invention is represented by the appended claims rather than the foregoing description, and all changes within the meaning and range of equivalents thereof are intended to be covered therein.

### Industrial Applicability

The pulse wave detector of the present invention is effective particularly in application to a portable blood pressure monitor or the like.

Although the present invention has been described with reference to the specific embodiment, the present invention is not limited to the embodiment, and various changes can be made without departing from the scope of the invention defined by the claims.

### Explanation of Reference Numerals

- 10, 11, 12, 13, 14, 15: biometric information measurement device
- 20, 20B: housing
- 20C: coupling portion
- 21: detection surface
- 22: pulse wave detecting section (detecting section)
- 23: display surface
- 26: second end portion
- 27: second engaging portion
- 28: first end portion
- 29: first engaging portion
- 31: hole portion
- 40: band
- 41: basal end portion
- 42, 42A, 45, 45A: hook and loop fastener
- 43: tip end portion
- 44: arbitrary position
- 100: body portion
- S: ulna
- T: radius
- TD: radial artery
- H: left hand
- W: wrist

## Claims

1. A pulse wave detector which is used while being attached to a wrist (W) of a measurement subject, the pulse wave detector comprising:
a body portion (20) which includes a detecting section (22) disposed in a position which is opposed to a radial artery (TD) of the measurement subject and configured to detect a pulse wave from the radial artery (TD);
a band (40) which is configured to be wound around the wrist (W) in a state where the band (40) is engaged with the body portion (20);
a basal-end engaging portion (29) which is disposed on a first end portion (28) of the body portion (20) in a circumferential direction of the wrist (W) and on a side of a surface (23) opposite to a detection surface (21) in the body portion (20), and with which a basal end portion (41) of the band (40) is engaged;
an arbitrary-position engaging portion (27) which is disposed on a second end portion (26) of the body portion (20) in the circumferential direction and on the detection surface (21), and with which an arbitrary position (44) between the basal end portion (41) engaged with the basal-end engaging portion (29) and a tip end portion (43) of the band (40) is engaged in a folded back state; and
engagement members (45) which are configured to detachably engage the tip end portion (43) with the band (40) in a state where the band (40) which is folded back in the arbitrary-position engaging portion (27) and the basal end portion (41) engaged with the basal-end engaging portion (29) overlap with each other,
wherein the basal-end engaging portion (29) is configured by a first member which is erected from the first end portion (28) of the body portion (20) and which has a Π-shape, and a first hole portion (31) is formed between the first member and the first end portion (28) of the body portion (20),
wherein the arbitrary-position engaging portion (27) is configured by a second member which is erected from the second end portion (26) of the body portion (20) and which has a Π-shape, and a second hole portion is formed between the second member and the second end portion (26) of the body portion (20), and
wherein the engagement members (45) are formed on surfaces of the basal end portion (41) and the tip end portion (43) of the band (40).

2. The pulse wave detector according to claim 1,
wherein the basal end portion (41) of the band (40) is detachably engaged with the basal-end engaging portion (29).

3. The pulse wave detector according to claim 2,
wherein the basal end portion (41) of the band (40) is detachably engaged in the basal-end engaging portion (29) in a state of being folded back in a direction to be separated from the first end portion (28).

4. The pulse wave detector according to any one of claims 1 to 3,
wherein the tip end portion (43) is engaged with the band (40) through the engagement member (45) in a state where a tip end of the tip end portion (43) is directed from a side of the detection surface (21) to a side of the surface opposite to the detection surface (21) in the body portion (20).

5. The pulse wave detector according to any one of claims 1 to 3,
wherein the first end portion (28) of the body portion (20) is an end portion on a side of an ulna of the wrist (W), and
wherein the second end portion (26) of the body portion (20) is an end portion on a side of a radius of the wrist (W).

6. The pulse wave detector according to any one of claims 1 to 5,
wherein the detecting section (22) is configured to detect a pressure pulse wave from the radial artery (TD) by using a pressure detecting element.

7. A biometric information measurement device (10) comprising:
the pulse wave detector according to any one of claims 1 to 6; and
a biometric information calculating section which is configured to calculate biometric information based on the pulse wave detected by the detecting section (22).

## Patentansprüche

1. Pulswellendetektor, der verwendet wird, während er an einem Handgelenk (W) einer Messperson angebracht ist, wobei der Pulswellendetektor umfasst:
einen Körperabschnitt (20), der einen Detektierungsabschnitt (22) aufweist, der an einer Position angeordnet ist, die einer Radialis-Arterie (TD) der Messperson gegenüberliegt, und dafür eingerichtet ist, eine Pulswelle von der Radialis-Arterie (TD) zu detektieren;
ein Band (40), das dafür eingerichtet ist, in einem Zustand, in dem das Band (40) mit dem Körperabschnitt (20) in Eingriff steht, um das Handgelenk (W) herum gelegt zu werden;
einen Basalende-Eingriffnahmeabschnitt (29), der an einem ersten Endabschnitt (28) des Körperabschnitts (20) in einer Umfangsrichtung des Handgelenks (W) und auf einer Seite einer Fläche (23) gegenüber einer Detektionsfläche (21) in dem Körperabschnitt (20) angeordnet ist, und mit dem ein basaler Endabschnitt (41) des Bandes (40) im Eingriff steht;
einen positionsbeliebigen Eingriffnahmeabschnitt (27), der an einem zweiten Endabschnitt (26) des Körperabschnitts (20) in der Umfangsrichtung und an der Detektionsfläche (21) angeordnet ist, und mit dem eine beliebige Position (44) zwischen dem basalen Endabschnitt (41), der mit dem Basalende-Eingriffnahmeabschnitt (29) im Eingriff steht, und einem Spitzenendabschnitt (43) des Bandes (40) in einem zurückgefalteten Zustand im Eingriff steht; und
Eingriffnahmeelemente (45), die dafür eingerichtet sind, den Spitzenendabschnitt (43) lösbar mit dem Band (40) in einem Zustand in Eingriff zu bringen, in dem das Band (40), das in den positionsbeliebigen Eingriffnahmeabschnitt (27) zurückgefaltet ist, und der basale Endabschnitt (41), der mit dem Basalende-Eingriffnahmeabschnitt (29) im Eingriff steht, einander überlappen,
wobei der Basalende-Eingriffnahmeabschnitt (29) von einem ersten Element gebilet ist, das von dem ersten Endabschnitt (28) des Körperabschnitts (20) aufgerichtet ist und das eine n-Form aufweist, und ein erster Lochabschnitt (31) zwischen dem ersten Element und dem ersten Endabschnitt (28) des Körperabschnitts (20) ausgebildet ist,
wobei der positionsbeliebige Eingriffnahmeabschnitt (27) von einem zweiten Element gebildet ist, das von dem zweiten Endabschnitt (26) des Körperabschnitts (20) aufgerichtet ist und das eine n-Form aufweist, und ein zweiter Lochabschnitt zwischen dem zweiten Element und dem zweiten Endabschnitt (26) des Körperabschnitts (20) ausgebildet ist, und
wobei die Eingriffnahmeelemente (45) auf Flächen des basalen Endabschnitts (41) und des Spitzenendabschnitts (43) des Bandes (40) ausgebildet sind.

2. Pulswellendetektor nach Anspruch 1,
wobei der basale Endabschnitt (41) des Bandes (40) lösbar mit dem Basalende-Eingriffnahmeabschnitt (29) im Eingriff steht.

3. Pulswellendetektor nach Anspruch 2,
wobei der basale Endabschnitt (41) des Bandes (40) in einem Zustand, in dem er in eine Richtung zurückgefaltet ist, in der er von dem ersten Endabschnitt (28) getrennt ist, lösbar mit den Basalende-Eingriffnahmeabschnitt (29) im Eingriff steht.

4. Pulswellendetektor nach einem der Ansprüche 1 bis 3, wobei der Spitzenendabschnitt (43) durch das Eingriffnahmeelement (45) mit dem Band (40) in einem Zustand im Eingriff steht, in dem ein Spitzenende des Spitzenendabschnitts (43) von einer Seite der Detektionsfläche (21) zu einer Seite der Fläche gegenüber der Detektionsfläche (21) in dem Körperabschnitt (20) gerichtet ist.

5. Pulswellendetektor nach einem der Ansprüche 1 bis 3, wobei der erste Endabschnitt (28) des Körperabschnitts (20) ein Endabschnitt auf einer Seite einer Elle des Handgelenks (W) ist, und
wobei der zweite Endabschnitt (26) des Körperabschnitts (20) ein Endabschnitt auf einer Seite einer Speiche des Handgelenks (W) ist.

6. Pulswellendetektor nach einem der Ansprüche 1 bis 5, wobei der Detektierungsabschnitt (22) dafür eingerichtet ist, eine Druckpulswelle von der Radialis-Arterie (TD) unter Verwendung eines Druckdetektierungselements zu detektieren.

7. Vorrichtung (10) zum Messen biometrischer Informationen, umfassend:
den Pulswellendetektor nach einem der Ansprüche 1 bis 6; und
einen Biometriedaten-Berechnungsabschnitt, der dafür eingerichtet ist, biometrische Informationen auf der Grundlage der durch den Detektierungsabschnitt (22) detektierten Pulswelle zu berechnen.

## Revendications

1. Détecteur d'ondes pulsatiles utilisé en étant attaché à un poignet (W) d'un sujet de mesure, le détecteur d'ondes pulsatiles comprenant :
une partie corps (20) qui comporte une section de détection (22) disposée dans une position opposée à une artère radiale (TD) du sujet de mesure et configurée pour détecter une onde pulsatile provenant de l'artère radiale (TD) ;
une bande (40) qui est configurée pour être enroulé autour du poignet (W) dans un état où la bande (40) est engagée avec la partie corps (20) ;
une partie d'engagement d'extrémité de base (29) qui est disposée sur une première partie d'extrémité (28) de la partie corps (20) dans une direction circonférentielle du poignet (W) et sur un côté d'une surface (23) opposée à une surface de détection (21) dans la partie corps (20), et avec laquelle une partie d'extrémité de base (41) de la bande (40) est engagée ;
une partie d'engagement à position arbitraire (27) qui est disposée sur une deuxième partie d'extrémité (26) de la partie corps (20) dans la direction circonférentielle et sur la surface de détection (21), et avec laquelle une position arbitraire (44) entre la partie d'extrémité de base (41) engagée avec la partie d'engagement d'extrémité de base (29) et une partie d'extrémité de pointe (43) de la bande (40) est engagée dans un état replié ; et
des éléments d'engagement (45) qui sont configurés pour engager de manière détachable la partie d'extrémité de pointe (43) avec la bande (40) dans un état où la bande (40) qui est repliée dans la partie d'engagement à position arbitraire (27) et la partie d'extrémité de base (41) engagée avec la partie d'engagement d'extrémité de base (29) se chevauchent l'une l'autre,
dans lequel la partie d'engagement d'extrémité de base (29) est configurée par un premier élément qui est érigé à partir de la première partie d'extrémité (28) de la partie corps (20) et qui a une forme en n, et une première partie trouée (31) est formée entre le premier élément et la première partie d'extrémité (28) de la partie corps (20),
dans lequel la partie d'engagement à position arbitraire (27) est configurée par un deuxième élément qui est érigé à partir de la deuxième partie d'extrémité (26) de la partie corps (20) et qui a une forme en n, et une deuxième partie trouée est formée entre le deuxième élément et la deuxième partie d'extrémité (26) de la partie corps (20), et
dans lequel les éléments d'engagement (45) sont formés sur les surfaces de la partie d'extrémité de base (41) et de la partie d'extrémité de pointe (43) de la bande (40).

2. Détecteur d'ondes pulsatiles selon la revendication 1, dans lequel la partie d'extrémité de base (41) de la bande (40) est engagée de manière détachable avec la partie d'engagement d'extrémité de base (29).

3. Détecteur d'ondes pulsatiles selon la revendication 2, dans lequel la partie d'extrémité de base (41) de la bande (40) est engagée de manière détachable dans la partie d'engagement d'extrémité de base (29) dans un état où elle est repliée dans une direction permettant de la séparer de la première partie d'extrémité (28).

4. Détecteur d'ondes pulsatiles selon l'une quelconque des revendications 1 à 3,
dans lequel la partie d'extrémité de pointe (43) est engagée avec la bande (40) par l'intermédiaire de l'élément d'engagement (45) dans un état où une extrémité de pointe de la partie d'extrémité de pointe (43) est dirigée d'un côté de la surface de détection (21) vers un côté de la surface opposée à la surface de détection (21) dans la partie corps (20).

5. Détecteur d'ondes pulsatiles selon l'une quelconque des revendications 1 à 3,
dans lequel la première partie d'extrémité (28) de la partie corps (20) est une partie d'extrémité sur un côté du cubitus du poignet (W), et
dans lequel la deuxième partie d'extrémité (26) de la partie corps (20) est une partie d'extrémité sur un côté du radius du poignet (W).

6. Détecteur d'ondes pulsatiles selon l'une quelconque des revendications 1 à 5,
dans lequel la section de détection (22) est configurée pour détecter une onde pulsatile de pression provenant de l'artère radiale (TD) à l'aide d'un élément de détection de pression.

7. Dispositif de mesure d'informations biométriques (10) comprenant :
le détecteur d'ondes pulsatiles selon l'une quelconque des revendications 1 à 6 ; et
une section de calcul d'informations biométriques configurée pour calculer des informations biométriques sur la base de l'onde pulsatile détectée par la section de détection (22).
